# EUROPEAN PATENT APPLICATION

(11) **EP 2 191 847 A1**
(43) Date of publication of application: **02.06.2010**
(21) Application number: 08020186.6
(22) Date of filing: 19.11.2008
(51) Int. Cl.: A61K 47/10, A61K 38/48

(54) **Method for preparing compositions comprising a protein, water and glycerol**

(71) Applicant: Merz Pharma GmbH & Co. KGaA, 60318 Frankfurt (DE)
(72) Inventor: Frevert, Jürgen, Dr., 10625 Berlin (DE)
(74) Representative: Ricker, Mathias

(57) **Abstract**

Method for preparing a composition comprising a clostridial neurotoxin, water and glycerol, comprising: adding said glycerol to said protein and water, wherein said glycerol contains less than 60 ppm by weight of said aldehyde based on the total amount of said glycerol.

## Description

### FIELD OF THE INVENTION

This invention provides a method for preparing stabilized compositions comprising a protein and water. The method comprises adding glycerol to said protein and water, wherein said glycerol contains less than 60 ppm by weight of an aldehyde, which may be contained within said glycerol. The method of the invention is particularly useful for preparing stabilized compositions of clostridial neurotoxins such as a botulinum neurotoxin in water having an improved biological activity.

### BACKGROUND OF THE INVENTION

Proteins are frequently obtained in aqueous dispersion or solution by chemical and biological pulping processes or fermentation processes. Moreover, the elucidation of the biophysical properties of a protein in solution is a key to understanding its biological activity. However, proteins in water may undergo unfolding transitions, which may change their structure that is required to generate a particular biological activity. This is a severe drawback since such a change in the structure may result in the reduction or even loss of said particular biological activity. If a protein is intended for use in a pharmaceutical composition or drug, a partially unfolded protein may even be not longer suitable for the manufacture of such pharmaceutical composition or drug or may lead to pharmaceutical compositions or drugs having a reduced biological activity and/or undesired side-effects.

It is believed that said change of the structure occurs due to the solvation properties of water. For stabilization of such proteins in order to suppress said unfolding transition, frequently glycerol is used. Glycerol alters the solvation properties of water to favor the native conformation of proteins. The mechanism by which this occurs may be based on the co-solvent exclusion and preferential hydration of the protein. However, it has also been discussed that the protein is dehydrated by glycerol, thereby maintaining the spatial structure.

In recent years, proteins from the class of clostridial neurotoxins such as botulinum neurotoxins have become the standard agent in the treatment of a disease or condition in a patient caused by or associated with hyperactive cholinergic innervation of muscles or an exocrine gland, wherein the neurotoxic component blocks acetylcholine secretion into the synaptic cleft. Examples of such diseases or conditions are focal dystonias and spastic indications.

Treatment of patients generally involves injection of a pharmaceutical composition comprising the neurotoxin and water into affected muscle tissue, bringing the agent near the neuromuscular end plate, i.e. close to the cellular receptor mediating its uptake into the nerve cell controlling said affected muscle. Various degrees of neurotoxin spread have been observed. This spread is thought to correlate with the injected amounts and the particular preparation of neurotoxin injected. Resulting from the spread, systematic side effects caused by the inhibition of acetylcholine release, may be observed at nearby muscle tissue. The incidents of unintended paralysis of untreated muscles can largely be avoided by reducing the injected doses to the therapeutically relevant level. Overdosing may also be a problem with regard to the patients' immune system, as the injected neurotoxin may trigger the formation of neutralizing antibodies. If this occurs, the toxin will be inactivated without being able to relieve the involuntary muscle activity.

Discrepancy on the dose equivalents or variations in the determined activity of preparations comprising botulinum neurotoxins such as available sales products or batches produced during the manufacturing process, commonly a fermentation process, poses an increased risk for patients through possible side effects and the development of immunity. Therefore, it is of crucial importance to keep the biological activity of clostridial neurotoxin contained in said sales products or production batches consistent (i.e. without significant variation) and as accurate as possible, in order to adjust the toxin concentration to a reliable effective dose for the benefit of the patient. This may also serve as an incentive to the manufacturers to offer formulations allowing optimum exploitation of biological activity for different therapeutic purposes.

Although it is known to stabilize aqueous formulations of proteins or fermentation broths containing proteins from which pharmaceutical preparations are prepared, using glycerol as described above, it has now been discovered that despite the presence of glycerol said proteins nevertheless may undergo changes in structure that renders them labile or even unsuitable for the manufacture of pharmaceutical compositions that should have a consistent and reliable biological activity in order to avoid the above addressed drawbacks in the application of proteins, particularly in the application of clostridial neurotoxins such as botulinum neurotoxins.

### OBJECTS OF THE INVENTION

It was an object of the invention to provide a method according to which a protein in water such as a clostridial neurotoxin protein may be stabilized in a manner such that the above addressed drawbacks can be reduced or even avoided.

### SUMMARY OF THE INVENTION

Surprisingly, it has been discovered that glycerol from different sources may exhibit different stabilization properties, thus differently affecting the biological activity of proteins in water. The term "source" encompasses the origin of the glycerol, e.g. the manufacturing method and/or its aging and/or storage conditions.

It has further been surprisingly discovered that glycerol obtained by the technically known processes such as saponification of vegetable oils in general exhibits better stabilization properties than synthetically prepared glycerol, e.g. glycerol obtained from the known hydration process of epichlorohydrin, or glycerol obtained in the transesterification process of vegetable oils with methanol. Freshly prepared glycerol exhibits in general better stabilization properties than stored respectively aged glycerol.

It has further been surprisingly discovered that the stabilization properties correlate with aldehyde quantities which may be contained in said glycerol. In general, synthetically prepared glycerol or glycerol obtained in said transesterification process contains higher quantities of aldehydes than glycerol obtained in said saponification process. Stored glycerol in general contains higher quantities of aldehydes than freshly produced glycerol due to oxidation processes that may degrade glycerol. By-products of the synthetic manufacturing process or from the degradation process are aldehydes such as formaldehyde, acrylaldehyde or glycerolaldehyde.

The above addressed drawbacks can be reduced or even avoided, if for the stabilization of a protein, in particular a clostridial neurotoxin such as a botulinum neurotoxin, in water, a glycerol is used that contains less than 60 ppm by weight of an aldehyde based on the total amount of glycerol.

Accordingly, the invention relates to a method for preparing a composition comprising a protein, preferably a clostridial neurotoxin, water and glycerol, comprising: contacting said glycerol with said protein and water, wherein said glycerol contains less than 60 ppm by weight of an aldehyde based on the total amount of said glycerol.

In one embodiment, the glycerol contains less than 40 ppm by weight of said aldehyde based on the total amount of said glycerol.

In another embodiment, the glycerol contains less than 20 ppm by weight of said aldehyde based on the total amount of said glycerol.

In still another embodiment, the glycerol contains less than 10 ppm by weight of said aldehyde based on the total amount of said glycerol.

In one embodiment, said clostridial neurotoxin is botulinum neurotoxin

In one embodiment, said botulinum neurotoxin is a serotype selected from the group consisting of A, B, C, D, E, F, and G.

In one embodiment, said botulinum neurotoxin is botulinum toxin of serotype A.

In another embodiment, said botulinum neurotoxin is a chemically or genetically modified derivative of a serotype selected from the group consisting of A, B, C, D, E, F, and G.

In another embodiment, the neurotoxin is free of complexing proteins.

In one embodiment, the amount of said glycerol is in the range of from 0.1 % by weight to 40 % by weight based on the total amount of said glycerol and water.

In one embodiment, the amount of said protein, preferably a clostridial neurotoxin, is in the range of from 0.1 to 30 % by weight based on the total amount of said glycerol.

The invention also relates to a composition comprising a clostridial neurotoxin, water and glycerol, wherein said glycerol contains less than 60 ppm by weight of an aldehyde based on the total amount of said glycerol.

In another embodiment, the invention relates to a composition comprising a clostridial neurotoxin, water and glycerol, wherein said glycerol contains less than 60 ppm by weight of an aldehyde based on the total amount of said glycerol, or to a composition prepared according to one of the methods of the invention, for use in the manufacture of a pharmaceutical composition.

In a further embodiment, said pharmaceutical composition is used for treating a disease or condition caused by or associated with hyperactive cholinergic innervation of muscles or exocrine glands in a patient.

In a further embodiment, the invention relates to a method for manufacturing a pharmaceutical composition, comprising: diluting a composition comprising a clostridial neurotoxin, water and glycerol, wherein said glycerol contains less than 60 ppm by weight of an aldehyde based on the total amount of said glycerol.

### DETAILED DESCRIPTION OF THE INVENTION

The present invention may be understood more readily by reference to the following detailed description and to preferred embodiments of the invention and the examples included therein.

Specifically, the invention relates to a method for preparing a composition comprising a protein, preferably a clostridial neurotoxin, water and glycerol, comprising:
contacting said glycerol with said protein and water, wherein said glycerol contains less than 60 ppm by weight of an aldehyde based on the total amount of said glycerol.

The term "less than 60 ppm by weight" encompasses a range of from 0.0 to less than 60 ppm by weight, or from 0.001 to less than 60 ppm by weight, or from 0.01 to less than 60 ppm by weight. Accordingly, the used glycerol may be free of any aldehyde.

In this context, 1 ppm by weight is equivalent to 1 part aldehyde based on 1,000,000 parts of glycerol, which corresponds to 1 mg aldehyde based on 1 kg glycerol.

In one embodiment, the glycerol contains less than 40 ppm by weight of said aldehyde based on the total amount of said glycerol.

In another embodiment, the glycerol contains less than 20 ppm by weight of said aldehyde based on the total amount of said glycerol.

In still another embodiment, the glycerol contains less than 10 ppm by weight of said aldehyde based on the total amount of said glycerol.

Therefore, in one embodiment, the amount of aldehyde being contained in said glycerol is determined prior to the contacting of said glycerol with said protein and water.

The term "protein" encompasses proteins, proteids such as glycoproteins, lipoproteins, nucleic proteins, and peptides.

The term "protein" further relates to any protein, that can be produced by cleaving cells of plants or animals or humans, such as chemical or biological pulping processes, or any protein that can be produced by fermentation processes.

In a specific embodiment, said protein is a clostridial neurotoxin.

The term "clostridial toxins" or "clostridial neurotoxin" encompasses clostridial toxin complexes as well as high purity neurotoxin, that is a neurotoxin preparation which is free of any other clostridial proteins.

By "botulinum toxin complex" should be understood a botulinum toxin associated with at least another non-toxic protein. As apparent, the term "botulinum toxin complex", as used herein, comprises the 300 kD, 450 kDa and the 900 kDa botulinum toxin complex, which is e.g. obtainable from cultures of *Clostridium* botulinum. Such preparations are commercially available e.g. by lpsen Ltd. (Dysport^{®}), Allergan (Botox^{®}) or Solstice Neurosciences, Inc. (botulinum toxin type B, Neurobloc^{®}). A high purity neurotoxin, free of any other clostridial proteins, is e.g. available from Merz Pharmaceuticals (Xeomin^{®}).

In one embodiment, said clostridial neurotoxin is botulinum neurotoxin.

In another embodiment, said botulinum neurotoxin is of a serotype selected from the group consisting of A, B, C, D, E, F, and G.

In another embodiment, said botulinum neurotoxin is a chemically or genetically modified derivative of a serotype selected from the group consisting of A, B, C, D, E, F, and G.

A chemically modified derivative of said neurotoxin may be one that is modified e.g. by pyruvation, phosphorylation, sulphatation, lipidation, glycosylation and/or pegylation. One or more amino acids of the neurotoxin may be chemically modified. Chemical modification may be beneficial to the uptake or stability of the neurotoxin.

In one embodiment, said chemical modification is lipidation. Modifying residues may be added to the neurotoxic component, e.g. by means of an enzymatic *in vitro* reaction or by using appropriate chemical reaction conditions. Alternatively, modifying enzymatic functions may be provided *in trans* by expressing the enzyme within the host cell.

A genetically modified derivative of said neurotoxin is one that has been modified by deletion, insertion or substitution of one or more amino acids contained in the proteins of said serotype. In one embodiment, said genetically modified neurotoxins contain 1, 2, 3, 4, 5, 6, 7, 8, 9, 10 or up to 20 amino acid mutations. Preferably, the mutation does not compromise the biological activity of the neurotoxins. However, it is also envisaged to use mutations to modulate the biological activity of the neurotoxic component.

A modified toxin preferably is biologically active.

A biologically active toxin is a toxin being capable to be uptaken into a cell, thereby proteolytically cleaving one or more polypeptides involved in the SNARE complex and thereby blocking the secretion of acetylcholine. Accordingly, toxin activity can be measured by an in vitro assay such as a SNAP-25 cleavage assay, or by an LD₅₀ assay, which are both known to the person skilled in the art.

In one embodiment, the botulinum toxin is botulinum toxin of serotype A. Botulinum toxin of serotype A is commercially available e.g. from Merz Pharmaceuticals (Xeomin^{®}). It is the drug of choice to improve several forms of focal dystonia.

In another embodiment, the neurotoxin is free of complexing proteins.

In another embodiment, the neurotoxin is formulated together with complexing proteins. Such formulations are e.g. available from lpsen Ltd. (Dysport^{®}) or Allergan Inc. (Botox^{®}).

The term "aldehyde" encompasses one or more aldehydes. Aldehydes are for example formaldehyde, acrylaldehyde or glycerolaldehyde.

In one embodiment, the type of aldehyde or aldehydes and the quantity thereof being contained in said glycerol are determined by gas-phase chromatography. Such a determination method is known in the art.

Glycerol that is suitable for practicing the invention may be obtained from the above addressed processes, i.e. saponification of vegetable oils, transesterification of vegetable oils or hydration of epichlorohydrin, provided said processes result in a product comprising less than 60 ppm by weight of said aldehyde based on the total amount of said glycerol.

Aldehydes being contained in said glycerol may be removed by methods that are basically known to the skilled person, e.g. by subjecting said glycerol to a vacuum treatment in the form of a degassing process, or by stripping said aldehydes with an inert gas such as nitrogen, until the level of aldehyde is less than 60 ppm by weight based on the total amount of glycerol.

In one embodiment, the thus purified glycerol is kept under inert gas such as nitrogen in order to avoid oxidation processes to aldehydes such as acrylaldehyde or glycerolaldehyde.

The term "contacting said glycerol with said protein and water" encompasses adding glycerol to a solution or dispersion of the protein in water, or vice versa, i.e. to add a solution or dispersion of the protein in water to glycerol; or adding the protein to a solution of glycerol in water, or vice versa; or suspending the protein in glycerol and to add water, or vice versa.

In one embodiment, glycerol is added to a solution or dispersion of the protein in water.

In general, the amount of said glycerol is in the range of from 0.1 % by weight to 40 % by weight based on the total amount of said glycerol and water.

However, it is also possible to employ glycerol in lower quantities such as 1 % by weight to 30 % by weight, or 2 % by weight to 30 % by weight, or 3 % by weight to 10 % by weight based on the total amount of said glycerol and water.

In general, the amount of said protein, preferably a clostridial neurotoxin, is in the range of from 0.1 % by weight to 30 % by weight based on the total amount of said glycerol.

According to the teaching of the present invention, the amount of said protein in the composition of the present invention may be in the range of up to 10 mg per ml, up to 20 mg per ml, up to 30 mg per ml, up to 50 mg per ml or even up to 100 mg per ml.

In one embodiment, said protein, preferably said clostridial neurotoxin such as a botulinum neurotoxin, is a protein that has been obtained in a fermentation process.

It is possible to isolate the protein from said fermentation process, e.g. by the known methods, e.g. by chromatographical methods, and to add the glycerol as described above.

However, it is also possible to add glycerol to the fermentation broth after the fermentation process has been terminated.

Furthermore, glycerol may be added at different steps of the harvesting and purification process of the neurotoxin.

In one embodiment, glycerol is added to the purified drug substance, i.e. to the active substance.

In one embodiment, glycerol is added as an excipient to the final formulation.

The method of the invention results in a composition comprising a clostridial neurotoxin, water and glycerol, that has an increased biological activity over such compositions that have been stabilized by glycerol having an aldehyde content of more than 60 ppm by weight based on the total amount of glycerol.

Accordingly, the invention also relates to a composition comprising a clostridial neurotoxin, water and glycerol, wherein said glycerol contains less than 60 ppm by weight of an aldehyde based on the total amount of said glycerol.

In another embodiment of the invention, said composition comprising a clostridial neurotoxin, water and glycerol, wherein said glycerol contains less than 60 ppm by weight of an aldehyde based on the total amount of said glycerol, is used for the manufacture of a pharmaceutical composition.

The term "pharmaceutical composition" encompasses a formulation in which an active ingredient for use as a medicament or diagnostic is contained or comprised. Such pharmaceutical composition may be suitable for diagnostic or therapeutic administration (i.e. by intramuscular or subcutaneous injection) to a human patient. The pharmaceutical composition may be lyophilized or vacuum dried, reconstituted, or in solution. When reconstituted it is preferred that the reconstituted solution is prepared adding sterile physiological saline (0.9 % NaCl).

Such composition may comprise additional components such as a pH buffer, excipient, diluent, cryoprotectant and/or stabilizer. "pH buffer" refers to a chemical substance being capable to adjust the pH value of a composition, solution and the like to a certain value or to a certain pH range.

The terms "stabilizing", stabilizes" or "stabilization" means that the active ingredient, i.e., the neurotoxic component in a reconstituted or aqueous solution pharmaceutical composition has greater than about 20 %, 30 %, 40 %, 50 %, 60 %, 70 %, 80 %, 90 %, and up to about 100 % of the toxicity that the biologically active neurotoxic component had prior to being incorporated into the pharmaceutical composition.

Examples of stabilizers are gelatin or albumin, preferably of human origin or obtained from a recombinant source. The stabilizers may be modified by chemical means or by recombinant genetics. In a preferred embodiment of the present invention, it is envisaged to use alcohols, e.g., inositol, mannitol, as cryoprotectant excipients to stabilize proteins during lyophilization.

In one embodiment of the present invention, the stabilizer may be a non-proteinaceous stabilizing agent comprising hyaluronic acid or poly(vinyl pyrrolidone) or poly(ethylene glycol) or a mixture of two or more thereof. Such composition is considered to be a safer composition possessing remarkable stability.

The term "cryoprotectant" refers to excipients, which may be added to the active ingredient, i.e., a neurotoxic component in a reconstituted or aqueous solution. Such a solution has greater than about 20 %, 30 %, 40 %, 50 %, 60 %, 70 %, 80 %, 90 %, and up to about 100 % of the toxicity that the biologically active neurotoxic component had prior to being freeze-dried in the pharmaceutical composition.

In another embodiment of the present invention, the pharmaceutical composition may comprise the neurotoxic component and a hyaluronic acid or a poly(vinyl pyrrolidone) or a poly(ethylene glycol), such composition being optionally pH stabilized by a suitable pH buffer, in particular by a sodium acetate buffer, and / or a cryoprotectant polyalcohol. Whether or not the pharmaceutical composition comprises, beside the neurotoxin component, additional components such as albumin, hyaluronic acid, a poly(vinyl pyrrolidone) and/or a poly(ethylene glycol) stabilizer, the pharmaceutical composition retains its potency substantially unchanged for six month, one year, two year, three year and/or four year periods when stored at a temperature between about +8 °C. and about -20 °C. Additionally, the indicated pharmaceutical compositions may have a potency or percent recovery of between about 20 % and about 100 % upon reconstitution.

A pharmaceutical composition within the scope of the present invention may include the neurotoxic component and one or more additional components. Preferably, the pharmaceutical compositions have a pH of between about 4 and 7.5 when reconstituted or upon injection, more preferably between about pH 6.8 and pH 7.6 and most preferably between pH 7.4 and pH 7.6. Generally, the pharmaceutical composition of the present invention comprises neurotoxic component in a quantity of about 6 pg to 30 ng, Preferably, the neurotoxic component has a biological activity of 50 to 500 LD50 units per ng neurotoxic component, as determined in a mouse LD50 assay. More preferably, the neurotoxic component has a biological activity of about 150 LD50. Such mouse assays are known in the art.

The pharmaceutical composition of the present invention may comprise a neurotoxin, and a hyaluronic acid. The hyaluronic acid stabilizes the neurotoxin. The pharmaceutical compositions disclosed herein may have a pH of between about 4 and 7.5 when reconstituted or upon injection. The hyaluronic acid in the instant pharmaceutical composition is preferably combined with the instant neurotoxic component in a quantity of 0.1 to 10 mg, especially 1 mg hyaluronic acid per ml in a 200 U/ml botulinum toxin solution. More preferably, the subject solution also contains a 1-100 mM, especially 10 mM sodium acetate buffer.

In another embodiment, the pharmaceutical composition may contain a polyalcohol as cryoprotectant. Examples of polyalcohols that might be used include, e.g., inositol, mannitol and other non-reducing alcohols.

In particular those embodiments of the present invention's pharmaceutical composition not comprising a proteinaceous stabilizer, preferably do not contain trehalose or maltotriose or related sugar or polyhydroxy compounds which are sometimes used as cryoprotectants. The poly(vinyl pyrrolidone) in the instant pharmaceutical composition is preferably combined with the instant neurotoxic component in a quantity of 10 to 500 mg, especially 100 mg poly(vinyl pyrrolidone) per ml in a 200 U/ml botulinum toxin solution. More preferably, the subject solution also contains a 1-100 mM, especially 10 mM sodium acetate buffer.

The poly(ethylene glycol) in the instant pharmaceutical composition is preferably combined with the instant neurotoxic component in a quantity of 10 to 500 mg, especially 100 mg poly(ethylene glycol) per ml in a 200 U/ml botulinum toxin solution. More preferably, the subject solution also contains a 1-100 m M, especially 10 mM sodium acetate buffer.

Thus, the instant invention encompasses a neurotoxic component formulated in a pharmaceutical composition, which contains a hyaluronic acid stabilizer or a poly(vinyl pyrrolidone) stabilizer or a poly(ethylene glycol) stabilizer. Additionally, the pharmaceutical composition may contain a sodium acetate buffer system and/or an alcoholic cryoprotectant.

In a specific embodiment of the invention, said pharmaceutical composition is used for treating a disease or condition caused by or associated with hyperactive cholinergic innervation of muscles or exocrine glands in a patient.

In one embodiment, said disease or condition caused by or associated with hyperactive cholinergic innervation of muscles or exocrine glands are focal dystonias and spastic indications.

In a further embodiment, said composition comprising a clostridial neurotoxin, water and glycerol, wherein said glycerol contains less than 60 ppm by weight of said aldehyde, is used as medicament.

In a further embodiment, it is also possible to further dilute a composition comprising a clostridial neurotoxin, water and glycerol, wherein said glycerol contains less than 60 ppm by weight of said aldehyde, i.e. a composition that e.g. has been obtained in a fermentation process, in order to obtain a pharmaceutical composition.

In one embodiment, for dilution, water and/or physiological saline (0.9 % NaCl) may be used.

Accordingly, the invention also relates to a method of preparing a pharmaceutical composition, comprising: diluting the composition prepared according to any one of the methods of the invention, or diluting a composition comprising a clostridial neurotoxin, water and glycerol, wherein said glycerol contains less than 60 ppm by weight of said aldehyde based on the total amount of said glycerol.

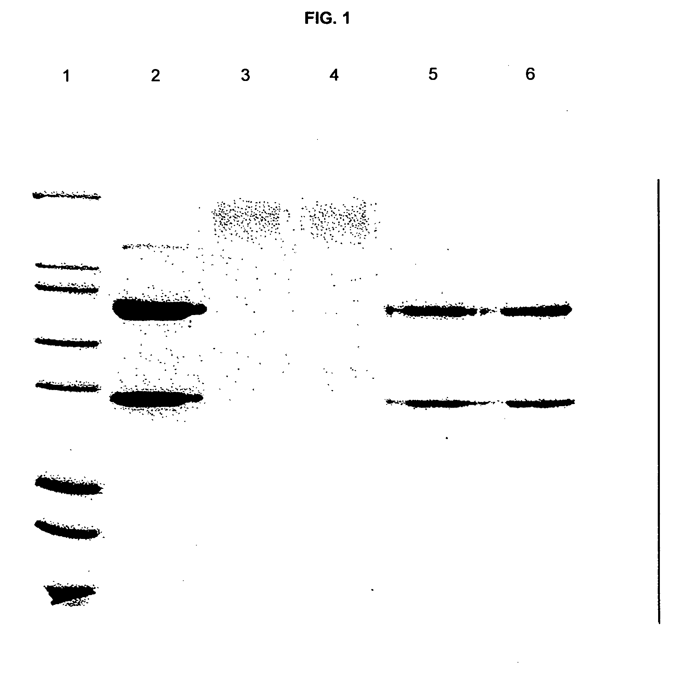

Figure 1 shows the result of a SDS-PAGE analysis of the composition of Example 1, a composition prepared with a glycerol containing 71 ppm aldehyde, and a composition of Example 2 according to the invention. SDS-PAGE is the abbreviation for sodium dodecylsulfate poly(acryl amide) gel electrophoresis. This method is well known in the art.

In Figure 1, the lanes 1 to 6 from left to right show in lane 1 a molecular weight marker, in lane 2 botulinum neurotoxin of serotype A, in lane 3 and 4 botulinum neurotoxin of serotype A stored at 40 °C according to Example 1, and in lane 5 and 6 botulinum neurotoxin of serotype A stored at 40 °C according to Example 2.

Whereas the sample from Example 2 showed the characteristic protein bands of the light chain (50 kD) and the heavy chain (100 kD), the sample from Example 1 only exhibited diffuse bands with a molecular weight > 200 kD.

Without wishing to be bound to a theory it is believed that due to the high amount of aldehyde being contained in the glycerol according to Example 1, crosslinking reactions of the protein with aldehyde occur that result in crosslinked neurotoxins having a higher molecular weight as compared to the non-crosslinked neurotoxins. These crosslinked proteins have a reduced biological activity as compared to the non-crosslinked proteins.

Due to the low amount of aldehyde in the glycerol according to Example 2, it is ensured that the amount and potency, i.e the biological activity, of the active agent is preserved and no undesired crosslinked neurotoxins are generated as may be seen in the glycerol-stabilized composition, in which the used glycerol has an aldehyde content of 71 ppm.

### EXAMPLES

### Example 1

A commercially available glycerol had a formaldehyde content of 71 ppm determined by gas-phase chromatography. 230 mg of said glycerol were added to 0.7 mL solution of 634 µg/mL botulinum toxin of serotype A in water. After storage at 40 °C for two weeks, said composition had an activity of < 10⁴ LD50 units/mg protein as determined in a mouse LD50 assay.

### Example 2

Example 1 was repeated with a glycerol having an formaldehyde content of less than 10 ppm. After storage at 40 °C for two weeks, said composition had an activity of 7.6 x 10⁷ LD50 units/mg protein as determined in a mouse LD50 assay.

## Claims

1. Method for preparing a composition comprising a clostridial neurotoxin, water and glycerol, comprising:
contacting said glycerol with said clostridial neurotoxin and water,
wherein said glycerol contains less than 60 ppm by weight of an aldehyde based on the total amount of said glycerol.

2. Method of claim 1, wherein the glycerol contains less than 40 ppm by weight of said aldehyde based on the total amount of said glycerol.

3. Method of claim 1 or 2, wherein the glycerol contains less than 20 ppm by weight of said aldehyde based on the total amount of said glycerol.

4. Method of any one of the preceding claims, wherein the glycerol contains less than 10 ppm by weight of said aldehyde based on the total amount of said glycerol.

5. Method of any one of the preceding claims, wherein said clostridial neurotoxin is botulinum toxin.

6. Method of claim 5, wherein said botulinum neurotoxin is of a serotype selected from the group consisting of A, B, C, D, E, F and G.

7. Method of claim 6, wherein the clostridial neurotoxin is botulinum toxin of serotype A.

8. Method of claim 6, wherein the botulinum neurotoxin is a chemically or genetically modified derivative of a botulinum neurotoxin serotype selected from the group consisting of A, B, C, D, E, F and G.

9. Method of any one of claims 5 to 8, wherein the neurotoxin is free of complexing proteins.

10. Method of any one of the preceding claims, wherein the amount of said glycerol is in the range of from 0.1 % by weight to 40 % by weight based on the total amount of said glycerol and water.

11. Method of any one of the preceding claims, wherein the amount of said clostridial neurotoxin is in the range of from 0.1 % by weight to 30 % by weight based on the total amount of said glycerol.

12. Composition comprising a clostridial neurotoxin, water and glycerol, wherein said glycerol contains less than 60 ppm by weight of an aldehyde based on the total amount of said glycerol.

13. Composition prepared according to any one of claims 1 to 11, or composition of claim 12, for use in the manufacture of a pharmaceutical composition.

14. Composition of claim 13, wherein said pharmaceutical composition is used for treating a disease or condition caused by or associated with hyperactive cholinergic innervation of muscles or exocrine glands in a patient.

15. Method of manufacturing a pharmaceutical composition, comprising:
diluting the composition according to claim 13.
